# EUROPEAN PATENT APPLICATION

(11) **EP 4 512 821 A1**
(43) Date of publication of application: **26.02.2025**
(21) Application number: 22942847.9
(22) Date of filing: 26.10.2022
(51) Int. Cl.: C07K 14/34, C12N 15/77, C12P 13/10

(54) **NOVEL GLUTAMATE GLUCONATE REPRESSOR VARIANT AND METHOD FOR PRODUCING L-ARGININE USING SAME**

(30) Priority: 18.05.2022 KR 20220061011
(71) Applicant: CJ CheilJedang Corporation, Seoul 04560 (KR)
(72) Inventor: KIM, Hyo Kyung, Seoul 04560 (KR); CHOI, Sun Hyoung, Seoul 04560 (KR); LEE, Zeewon, Seoul 04560 (KR); SHIM, Jihyun, Seoul 04560 (KR)
(74) Representative: Meissner Bolte Partnerschaft mbB
(86) International application number: PCT/KR2022/016417
(87) International publication number: WO 2023/224183

(57) **Abstract**

Provided are a novel gluconate repressor variant, a polynucleotide comprising the variant of the present disclosure, a microorganism of the genus *Corynebacterium* comprising the variant or polynucleotide of the present disclosure, and a method of producing L-arginine using the microorganism of the present disclosure.

## Description

### [Technical Field]

The present disclosure relates to a novel gluconate repressor variant, a polynucleotide comprising the variant of the present disclosure, a microorganism of the genus *Corynebacterium* comprising the variant of the present disclosure or the polynucleotide of the present disclosure, and a method of producing L-arginine using the microorganism of the present disclosure.

### [Background Art]

L-arginine is used for medicinal purposes such as liver function enhancers, brain function enhancers, multi-amino acid preparations, etc., and is also a substance that has recently received attention in food applications such as fish cake additives, health beverage additives, salt substitutes for patients with hypertension, etc. In order to produce high-concentration industrially applicable arginine, the use of microorganisms has been continuously studied.

Meanwhile, microorganisms of the genus *Corynebacterium,* particularly, *Corynebacterium glutamicum,* are gram-positive microorganisms widely used in the production of L-amino acids. For the production of L-arginine, target material-specific approaches are mainly used, such as a method of increasing the expression of a gene encoding an enzyme mainly involved in L-arginine synthesis in a strain of the genus *Corynebacterium,* or a method of deleting a gene unnecessary for L-arginine biosynthesis (Korean Patent No. 10-1102263).

However, there is still a growing need for research on methods capable of efficiently producing L-arginine with a high yield.

### [Disclosure]

### [Technical Problem]

The present inventors have developed a novel gluconate repressor variant for increasing L-arginine production, a polynucleotide encoding the variant, a microorganism for producing L-arginine, the microorganism comprising the variant or the polynucleotide, and a method of producing L-arginine using the microorganism, thereby completing the present disclosure.

### [Technical Solution]

An object of the present disclosure is to provide a gluconate repressor variant in which one or more amino acids corresponding to positions of an amino acid sequence of SEQ ID NO: 1 are substituted with different amino acids.

Another object of the present disclosure is to provide a polynucleotide encoding the variant of the present disclosure.

Still another object of the present disclosure is to provide a microorganism of the genus *Corynebacterium* comprising the variant of the present disclosure or the polynucleotide encoding the variant of the present disclosure.

Still another object of the present disclosure is to provide a microorganism of the genus *Corynebacterium* comprising a gluconate repressor variant, in which an amino acid corresponding to position 70 of an amino acid sequence of SEQ ID NO: 1 is substituted with a different amino acid, or a polynucleotide encoding the variant of the present disclosure.

Still another object of the present disclosure is to provide a method of producing L-arginine, the method comprising the step of culturing the microorganism of the present disclosure in a medium.

Still another object of the present disclosure is to provide a composition for producing L-arginine, the composition comprising the microorganism of the present disclosure, a medium in which the microorganism of the present disclosure is cultured, or a combination of two or more thereof.

Still another object of the present disclosure is to provide use of the microorganism of the present disclosure in producing L-arginine.

Still another object of the present disclosure is to provide use of a gluconate repressor variant in producing L-arginine, the gluconate repressor variant having substitutions of one or more amino acids selected from the group consisting of an amino acid corresponding to position 36, an amino acid corresponding to position 59, an amino acid corresponding to position 60, an amino acid corresponding to position 63, an amino acid corresponding to position 79, and an amino acid corresponding to position 92 of an amino acid sequence of SEQ ID NO: 1 with different amino acids.

### [Advantageous Effects]

When a microorganism of the genus *Corynebacterium* comprising a gluconate repressor variant of the present disclosure is cultured, it is possible to produce L-arginine with a high yield, as compared to using a microorganism without the variant.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a gluconate repressor variant in which one or more amino acids corresponding to positions of an amino acid sequence of SEQ ID NO: 1 are substituted with different amino acids.

As used herein, the term "gluconate repressor variant" refers to a gluconate repressor variant comprising substitutions of one or more amino acids in an amino acid sequence of a polypeptide having gluconate repressor activity.

As used herein, the term "gluconate repressor (GntR)" refers to a regulatory protein involved in gluconate metabolism or sugar uptake. The gluconate repressor may be used interchangeably with the term "gluconate repressor protein".

The gluconate repressor protein of the present disclosure may be, but is not particularly limited to, a gluconate repressor protein derived from a microorganism of the genus *Corynebacterium,* specifically, *Corynebacterium glutamicum,* or a variant thereof, but is not limited thereto. Specifically, the gluconate repressor protein may comprise, for example, the amino acid sequence of SEQ ID NO: 1, or an amino acid sequence having 70% or more homology or identity thereto, but is not limited thereto, as long as it has the gluconate repressor activity. Specifically, the amino acid sequence may comprise SEQ ID NO: 1 or an amino acid sequence having at least 70%, 80%, 85%, 90%, 95%, 96%, 97%, 98%, or 99% or more homology or identity to SEQ ID NO: 1. The sequence of SEQ ID NO: 1 may be obtained from NCBI's GenBank or Kyoto Encyclopedia of Genes and Genomes (KEGG), which is a known database. For example, it may be derived from the genus *Corynebacterium* or *Corynebacterium glutamicum,* more specifically, may be a polypeptide/protein comprising the amino acid sequence represented by SEQ ID NO: 1, but is not limited thereto. It is also apparent that an accessory protein having an amino acid sequence with deletion, modification, substitution, or addition of some sequence also falls within the scope of the present disclosure as long as the amino acid sequence has such homology or identity and exhibits efficacy corresponding to that of the protein.

Further, the gluconate repressor protein having the amino acid sequence of SEQ ID NO: 1 may be encoded by a polynucleotide having or comprising a sequence of SEQ ID NO: 2 or a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, or consisting of or essentially consisting of the sequence of SEQ ID NO: 2 or a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, but is not limited thereto.

As used herein, the term "variant" refers to a polypeptide in which one or more amino acids are conservatively substituted and/or modified so as to be different from the amino acid sequence of the variant before modification, while retaining functions or properties thereof. Such a variant may generally be identified by modifying one or more amino acids in the amino acid sequence of the polypeptide and evaluating the properties of the modified polypeptide. In other words, the ability of the variant may be increased, unchanged, or decreased, as compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "variant" may also be used interchangeably with a modification, modified polypeptide, modified protein, mutant, mutein, divergent, etc., and any term is not limited, as long as it is used in a sense of being mutated.

The variant may also comprise deletion or addition of amino acids that have minimal influence on the properties and secondary structure of the polypeptide. For example, a signal (or leader) sequence which co-translationally or post-translationally directs transfer of the protein may be conjugated at the N-terminus of the variant. The variant may also be conjugated to other sequence or a linker for identification, purification, or synthesis.

The gluconate repressor variant of the present disclosure may be a gluconate repressor variant in which one or more amino acids selected from the group consisting of an amino acid corresponding to position 36, an amino acid corresponding to position 59, an amino acid corresponding to position 60, an amino acid corresponding to position 63, an amino acid corresponding to position 79, and an amino acid corresponding to position 92 of the amino acid sequence of SEQ ID NO: 1 are substituted with different amino acids, but is not limited thereto.

In one embodiment, the variant may further have a substitution of an amino acid corresponding to position 70 of the amino acid sequence of SEQ ID NO: 1 with a different amino acid, in addition to the substitutions of one or more amino acids selected from the group consisting of the amino acid corresponding to position 36, the amino acid corresponding to position 59, the amino acid corresponding to position 60, the amino acid corresponding to position 63, the amino acid corresponding to position 79, and the amino acid corresponding to position 92 of the amino acid sequence of SEQ ID NO: 1 with different amino acids, but is not limited thereto.

The "different amino acid" is not limited as long as it is an amino acid different from the amino acid before substitution. Meanwhile, when it is expressed that 'a specific amino acid has been substituted', it is obvious that the amino acid is substituted with an amino acid different from the amino acid before substitution, even though it is not specifically stated that the amino acid has been substituted with a different amino acid.

Amino acids may generally be classified based on similarity of polarity, charge, solubility, hydrophobicity, hydrophilicity, and/or amphipathic nature of residues.

Examples of this classification may comprise positively charged (basic) amino acids such as arginine, lysine, and histidine; negatively charged (acidic) amino acids such as glutamic acid and aspartic acid; amino acids with non-polar side chains (non-polar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, aspartic acid, and amino acids with uncharged side chains (uncharged amino acids also known as neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto.

For example, when it is expressed that "an amino acid corresponding to position 36 of SEQ ID NO: 1 is substituted with a different amino acid", it may mean that the amino acid is substituted with asparagine, phenylalanine, glycine, alanine, arginine, aspartate, cysteine, glutamic acid (glutamate), leucine, glutamine, histidine, proline, serine, tyrosine, lysine, tryptophan, valine, methionine, or threonine, excluding isoleucine which is the amino acid corresponding to position 36 of SEQ ID NO: 1, but is not limited thereto.

In the present disclosure, although being described as "a protein having an amino acid sequence represented by a specific SEQ ID NO.", it is obvious that any protein having an amino acid sequence with deletion, modification, substitution, conservative substitution, or addition of some sequence may be used in the present disclosure as long as the protein has activity identical or corresponding to that of the protein consisting of the amino acid sequence of the corresponding SEQ ID NO. For example, as long as a protein has activity identical or corresponding to that of the variant protein, it does not exclude addition of a sequence, which does not alter the function of the protein, upstream or downstream of the amino acid sequence, a naturally occurring mutation, a silent mutation thereof, or conservative substitution, and it is obvious that such a sequence addition or mutation also falls within the scope of the present disclosure.

As used herein, the "position N" may comprise position N and an amino acid position corresponding to position N. Specifically, it may comprise an amino acid position corresponding to any amino acid residue in a mature polypeptide disclosed in a particular amino acid sequence. The particular amino acid sequence may be the amino acid sequence of SEQ ID NO: 1.

As used herein, the term "corresponding to" refers to an amino acid residue at a position listed in a polypeptide, or an amino acid residue that is similar, identical, or homologous to a residue listed in a polypeptide. Confirming the amino acid at the corresponding position may involve determining a specific amino acid in a sequence that refers to a specific sequence. The "corresponding region" used herein generally refers to a similar or corresponding position in a related protein or reference protein.

For example, any amino acid sequence is aligned with SEQ ID NO: 1, and based on this, each amino acid residue of the amino acid sequence may be numbered by reference to the numerical position of the amino acid residue corresponding to the amino acid residue of SEQ ID NO: 1. For example, a sequence alignment algorithm such as that described herein may confirm the position of an amino acid, or a position where a modification such as substitution, insertion or deletion occurs, compared to a query sequence (also referred to as a "reference sequence").

For such alignment, for example, Needleman-Wunsch algorithm (Needleman and Wunsch, 1970, J. Mol. Biol. 48: 443-453), Needleman program of EMBOSS package (EMBOSS: The European Molecular Biology Open Software Suite, Rice et al., 2000), Trends Genet. 16: 276-277) and the like may be used without being limited thereto, and sequence alignment programs, pairwise sequence comparison algorithm and the like known in the art may be appropriately used.

In one embodiment, the variant provided in the present disclosure may comprise an amino acid sequence in which one, two, three, four, five, or six amino acids of positions corresponding to positions 36, 59, 60, 63, 79, and 92 from the N-terminus of SEQ ID NO: 1 are substituted with different amino acid(s).

In any one embodiment of the above-described embodiments, the variant may comprise one or more substitutions selected from the group consisting of a substitution of the amino acid corresponding to position 36 with asparagine, a substitution of the amino acid corresponding to position 59 with alanine, a substitution of the amino acid corresponding to position 60 with leucine, a substitution of the amino acid corresponding to position 63 with alanine, a substitution of the amino acid corresponding to position 79 with alanine, and a substitution of the amino acid corresponding to position 92 with leucine with regard to the amino acid sequence of SEQ ID NO: 1, but is not limited thereto.

In any one embodiment of the above-described embodiments, the variant may comprise one or more substitutions selected from the group consisting of the substitution of the amino acid corresponding to position 36 with asparagine, the substitution of the amino acid corresponding to position 59 with alanine, the substitution of the amino acid corresponding to position 60 with leucine, the substitution of the amino acid corresponding to position 63 with alanine, the substitution of the amino acid corresponding to position 79 with alanine, and the substitution of the amino acid corresponding to position 92 with leucine with regard to the amino acid sequence of SEQ ID NO: 1, and may further a substitution of the amino acid corresponding to position 70 with lysine with regard to the amino acid sequence of SEQ ID NO: 1, but is not limited thereto.

In any one embodiment of the above-described embodiments, the variant provided in the present disclosure may comprise a substitution of the amino acid corresponding to position 36 from the N-terminus of SEQ ID NO: 1 with a different amino acid.

In any one embodiment of the above-described embodiments, with regard to the variant, the amino acid corresponding to position 36 from the N-terminus of SEQ ID NO: 1 may be substituted with an amino acid selected from serine, cysteine, tyrosine, asparagine, and glutamine, which are polar amino acids. In any one embodiment of the above-described embodiments, the variant may be a variant in which the amino acid corresponding to position 36 from the N-terminus of SEQ ID NO: 1 is substituted with asparagine (N).

In any one embodiment of the above-described embodiments, the variant provided in the present disclosure may comprise a substitution of the amino acid corresponding to position 59 from the N-terminus of SEQ ID NO: 1 with a different amino acid.

In any one embodiment of the above-described embodiments, with regard to the variant, the amino acid corresponding to position 59 from the N-terminus of SEQ ID NO: 1 may be substituted with a non-polar amino acid. For example, the amino acid may be an amino acid selected from glycine, alanine, valine, leucine, isoleucine, methionine, phenylalanine, tryptophan, and proline. In any one embodiment of the above-described embodiments, the variant may be a variant in which the amino acid corresponding to position 59 from the N-terminus of SEQ ID NO: 1 is substituted with alanine (A).

In any one embodiment of the above-described embodiments, the variant provided in the present disclosure may comprise a substitution of the amino acid corresponding to position 60 from the N-terminus of SEQ ID NO: 1 with a different amino acid.

In any one embodiment of the above-described embodiments, with regard to the variant, the amino acid corresponding to position 60 from the N-terminus of SEQ ID NO: 1 may be substituted with a non-polar amino acid. For example, the amino acid may be an amino acid selected from glycine, alanine, leucine, isoleucine, methionine, phenylalanine, tryptophan, and proline. In any one embodiment of the above-described embodiments, the variant may be a variant in which the amino acid corresponding to position 60 from the N-terminus of SEQ ID NO: 1 is substituted with leucine (L).

In any one embodiment of the above-described embodiments, the variant provided in the present disclosure may comprise a substitution of the amino acid corresponding to position 63 from the N-terminus of SEQ ID NO: 1 with a different amino acid.

In any one embodiment of the above-described embodiments, with regard to the variant, the amino acid corresponding to position 63 from the N-terminus of SEQ ID NO: 1 may be substituted with a non-polar amino acid. For example, the amino acid may be an amino acid selected from glycine, alanine, valine, leucine, isoleucine, methionine, phenylalanine, tryptophan, and proline. In any one embodiment of the above-described embodiments, the variant may be a variant in which the amino acid corresponding to position 63 from the N-terminus of SEQ ID NO: 1 is substituted with alanine (A).

In any one embodiment of the above-described embodiments, the variant provided in the present disclosure may comprise a substitution of the amino acid corresponding to position 79 from the N-terminus of SEQ ID NO: 1 with a different amino acid.

In any one embodiment of the above-described embodiments, with regard to the variant, the amino acid corresponding to position 79 from the N-terminus of SEQ ID NO: 1 may be substituted with a non-polar amino acid. For example, the amino acid may be selected from the group consisting of glycine, alanine, valine, leucine, isoleucine, methionine, phenylalanine, tryptophan, and proline. In any one embodiment of the above-described embodiments, the variant may be a variant in which the amino acid corresponding to position 79 from the N-terminus of SEQ ID NO: 1 is substituted with alanine (A).

In any one embodiment of the above-described embodiments, the variant provided in the present disclosure may comprise a substitution of the amino acid corresponding to position 92 from the N-terminus of SEQ ID NO: 1 with a different amino acid.

In any one embodiment of the above-described embodiments, with regard to the variant, the amino acid corresponding to position 92 from the N-terminus of SEQ ID NO: 1 may be substituted with a non-polar amino acid. For example, the amino acid may be selected from the group consisting of glycine, alanine, leucine, isoleucine, methionine, phenylalanine, tryptophan, and proline. In any one embodiment of the above-described embodiments, the variant may be a variant in which the amino acid corresponding to position 92 from the N-terminus of SEQ ID NO: 1 is substituted with leucine (L).

In any one embodiment of the above-described embodiments, the variant provided in the present disclosure may comprise a substitution of the amino acid corresponding to position 70 from the N-terminus of SEQ ID NO: 1 with a different amino acid.

In any one embodiment of the above-described embodiments, with regard to the variant provided in the present disclosure, the amino acid corresponding to position 70 from the N-terminus of SEQ ID NO: 1 may be substituted with an amino acid selected from arginine, lysine, histidine, glutamic acid, and aspartic acid which are amino acids having with electrically charged side chains. For example, the amino acid may be selected from the group consisting of arginine, lysine, and histidine, which are basic amino acids. In any one embodiment of the above-described embodiments, the variant may be a variant in which the amino acid corresponding to position 70 from the N-terminus of SEQ ID NO: 1 is substituted with lysine (K).

Meanwhile, through sequence alignment known in the art, those skilled in the art may recognize, in any amino acid sequence, the amino acid corresponding to position 35, the amino acid corresponding to position 59, the amino acid corresponding to position 60, the amino acid corresponding to position 63, the amino acid corresponding to position 70, the amino acid corresponding to position 79, and the amino acid corresponding to position 92 of the amino acid sequence of SEQ ID NO: 1 of the present disclosure, and even though not separately described in the present disclosure, when "an amino acid at a specific position of a specific SEQ ID NO." is described, it is obvious that the amino acid comprises "an amino acid at position corresponding thereto" in any amino acid sequence.

Further, the variant of the present disclosure may comprise substitutions of one or more amino acids selected from the group consisting of amino acids corresponding to positions 36, 59, 60, 63, 79, and 92 from the N-terminus of SEQ ID NO: 1 with different amino acids, in an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1, or may further comprise a substitution of the amino acid corresponding to position 70 of the amino acid sequence of SEQ ID NO: 1 with a different amino acid, in addition to the substitutions of one or more amino acids selected from the group consisting of amino acids corresponding to positions 36, 59, 60, 63, 79, and 92 from the N-terminus of SEQ ID NO: 1 with different amino acids. It is also apparent that a variant having an amino acid sequence with deletion, modification, substitution, conservative substitution, or addition of some sequence also falls within the scope of the present disclosure as long as the amino acid sequence has such homology or identity and exhibits efficacy corresponding to that of the variant of the present disclosure.

For example, the variant of the present disclosure may have and/or comprise an amino acid sequence selected from the group consisting of amino acid sequences represented by SEQ ID NO: 23 to SEQ ID NO: 29, or may essentially consist of the amino acid sequence. Alternatively, the variant may comprise an amino acid sequence having at least 70%, 75%, 80%, 85%, 90%, 95%, 96%, 97%, 98%, 99%, 99.5%, 99.7%, or 99.9% or more homology or identity to an amino acid sequence selected from the group consisting of the amino acid sequences represented by SEQ ID NO: 23 to SEQ ID NO: 29.

For example, there are cases of having addition or deletion of a sequence which does not alter the function of the variant of the present disclosure at the N-terminus of, C-terminus of, and/or inside the amino acid sequence, a naturally occurring mutation, a silent mutation thereof, or a conservative substitution.

As used herein, the term "conservative substitution" means substituting an amino acid with another amino acid having similar structural and/or chemical properties. Such amino acid substitution may generally occur based on similarity in the polarity, charge, solubility, hydrophobicity, hydrophilicity, and/or amphipathic nature of the residue. For example, positively charged (basic) amino acids comprise arginine, lysine, and histidine; negatively charged (acidic) amino acids comprise glutamic acid and aspartate; aromatic amino acids comprise phenylalanine, tryptophan, and tyrosine; and hydrophobic amino acids comprise alanine, valine, isoleucine, leucine, methionine, phenylalanine, tyrosine, and tryptophan. Further, amino acids may be classified into amino acids with electrically charged side chains and amino acids with uncharged side chains. The amino acids with electrically charged side chains comprise aspartic acid, glutamic acid, lysine, arginine, and histidine, and the amino acids with uncharged side chains may be further classified into non-polar amino acids or polar amino acids. The non-polar amino acids comprise glycine, alanine, valine, leucine, isoleucine, methionine, phenylalanine, tryptophan, and proline, and polar amino acids comprise serine, threonine, cysteine, tyrosine, asparagine, and glutamine. Commonly, the conservative substitution may have little or no effect on the activity of a produced polypeptide. Commonly, the conservative substitution may have little or no effect on the activity of a protein or polypeptide.

Further, the variant may also comprise deletion or addition of amino acids that have minimal influence on the properties and secondary structure of the polypeptide. For example, a polypeptide may be conjugated to a signal (or leader) sequence at the N-terminus of the protein, which co-translationally or post-translationally directs the transfer of the protein. The polypeptide may also be conjugated to another sequence or a linker for identification, purification, or synthesis of the polypeptide.

For example, the variant of the present disclosure may have the gluconate repressor activity. Further, the variant of the present disclosure may have the activity to increase the L-arginine-producing ability, as compared to the wild-type polypeptide with the gluconate repressor activity.

Another aspect of the present disclosure provides a polynucleotide encoding the variant of the present disclosure.

As used herein, the term "polynucleotide" refers to a DNA or RNA strand having a certain length or more as a polymer of nucleotides in which nucleotide monomers are connected in a long chain by covalent bonds, and more specifically, refers to a polynucleotide fragment encoding the variant.

The polynucleotide encoding the gluconate repressor variant of the present disclosure may comprise any polynucleotide sequence without limitation, as long as it is a polynucleotide sequence encoding the gluconate repressor variant of the present disclosure. For example, the polynucleotide encoding the gluconate repressor variant of the present disclosure may be a polynucleotide sequence encoding the amino acid sequence of the variant of the present disclosure, but is not limited thereto.

For example, the polynucleotide encoding the gluconate repressor variant of the present disclosure may be, for example, a polynucleotide sequence selected from the group consisting of polynucleotide sequences represented by SEQ ID NO: 30 to SEQ ID NO: 36, or a polynucleotide sequence having at least 70%, 80%, 85%, 90%, 95%, 96%, 97%, 98%, or 99% or more homology or identity to a polynucleotide sequence selected from the group consisting of polynucleotide sequences represented by SEQ ID NO: 30 to SEQ ID NO: 36, but is not limited thereto. The nucleotide sequence having homology or identity may be those in the above range, excluding a sequence having 100% identity, or may be a sequence having less than 100% identity.

The polynucleotide of the present disclosure may undergo various modifications in the coding region without changing the amino acid sequence of the variant of the present disclosure in consideration of codon degeneracy or the codons preferred in an organism in which the variant of the present disclosure is to be expressed. Therefore, it is apparent that a polynucleotide which may be translated into a polypeptide consisting of the amino acid sequence of the variant of the present disclosure or a polypeptide having homology or identity thereto due to codon degeneracy may also be comprised.

Further, the polynucleotide of the present disclosure may comprise a probe that may be prepared from a known gene sequence, for example, may comprise any sequence without limitation as long as it is a sequence that is able to hybridize with a complementary sequence to the entirety or a part of the polynucleotide sequence of the present disclosure under stringent conditions.

The "stringent conditions" refers to conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, conditions in which polynucleotides having higher homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or conditions in which washing is performed once, specifically, twice to three times at a salt concentration and temperature equivalent to 60°C, 1XSSC, 0.1% SDS, specifically 60°C, 0.1XSSC, 0.1% SDS, more specifically 68°C, 0.1XSSC, 0.1% SDS, which are washing conditions for common Southern hybridization, may be listed.

Hybridization requires that two nucleic acids have complementary sequences, although mismatches between bases are allowed depending on the stringency of hybridization. The term "complementary" is used to describe the relationship between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "homology" or "identity" refers to a degree of similarity between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program to be used may be used together. Substantially, homologous or identical sequences may generally hybridize under moderately or highly stringent conditions to the entirety or a part of the sequence. It is apparent that hybridization also comprises hybridization with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387(1984)), BLASTP, BLASTN, and FASTA (Atschul, [S.] [F.,] [ETAL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al.(1970), J Mol Biol. 48:443, as disclosed by Smith and Waterman, Adv. Appl. Math(1981) 2:482. Briefly, the GAP program defines the same as the value obtained by dividing the number of similarly aligned symbols (i.e., nucleotides or amino acids) by the total number of the symbols in the shorter of the two sequences. The default parameters for the GAP program may comprise: (1) a binary comparison matrix (comprising a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358(1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps. Therefore, the term "homology" or "identity", as used herein, represents relevance between sequences.

Still another aspect of the present disclosure provides a vector comprising the polynucleotide of the present disclosure. The vector may be an expression vector for expressing the polynucleotide in microorganisms, but is not limited thereto.

As used herein, the term "vector" may comprise a DNA construct comprising a nucleotide sequence of a polynucleotide encoding a desired polypeptide which is operably linked to a suitable expression regulatory region (or expression control sequence) so that the desired polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable microorganism, and then replicate or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a microorganism so that the polypeptide encoded by the polynucleotide may be expressed in the microorganism. The transformed polynucleotide may be located regardless of the position, either by being inserted into the chromosome of the microorganism or located outside the chromosome as long as it may be expressed in the microorganism. Further, the polynucleotide comprises DNA and/or RNA encoding a desired polypeptide. The polynucleotide may be introduced in any form as long as it may be introduced into a microorganism and expressed. For example, the polynucleotide may be introduced into a microorganism in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a microorganism in its own form and operably linked to a sequence required for expression in the microorganism, but is not limited thereto.

As used herein, the term "operably linked" means that the polynucleotide sequence is functionally linked to a promoter sequence that initiates and mediates transcription of the polynucleotide encoding the desired variant of the present disclosure.

Still another aspect of the present disclosure provides a microorganism of the genus *Corynebacterium* comprising the gluconate repressor variant of the present disclosure, the polynucleotide encoding the gluconate repressor variant, or the vector comprising the polynucleotide.

For another example of the present disclosure, the microorganism of the present disclosure may be a microorganism of the genus *Corynebacterium* comprising the gluconate repressor variant in which the amino acid corresponding to position 70 of the amino acid sequence of SEQ ID NO: 1 is substituted with a different amino acid, a polynucleotide encoding the variant, or a vector comprising the polynucleotide.

The microorganism of the genus *Corynebacterium* of the present disclosure may be a microorganism having the gluconate repressor variant of the present disclosure or L-arginine-producing ability.

Specifically, the microorganism of the present disclosure may be a microorganism naturally having the gluconate repressor or the L-arginine-producing ability, or a microorganism prepared by introducing the variant of the present disclosure or the polynucleotide encoding the same (or the vector comprising the polynucleotide) into a parent strain without the gluconate repressor or the L-arginine-producing ability, and/or by providing the L-arginine-producing ability therefor, but is not limited thereto.

As used herein, the term "L-arginine" refers to an L-amino acid having the chemical formula of C₆H₁₄N₄O₂, which is a conditionally essential amino acid present in all organisms. L-arginine is known to be mainly produced by microorganisms of the genus *Corynebacterium* among microorganisms; however, it is known that these are subject to feedback inhibition by intracellular arginine (Vehary Sakanyan, et al, Microbiology, 142:9-108, 1996). Therefore, these microorganisms are known to have a limit in producing L-arginine with high yields.

As used herein, the term "microorganism (or strain)" comprises all wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism comprising genetic modification for production of a desired polypeptide, protein, or product.

As used herein, the term "microorganism having the L-arginine-producing ability" refers to a prokaryotic or eukaryotic microbial strain capable of producing L-arginine within a living organism, and may comprise both a microorganism prepared by providing the L-arginine-producing ability for a parent strain without the L-arginine-producing ability, or a microorganism that inherently has the L-arginine-producing ability. The L-arginine-producing ability may be conferred or enhanced by species improvement.

As used herein, the term "unmodified microorganism" does not exclude strains comprising mutations that may occur naturally in microorganisms, and may refer to a wild-type strain or a natural strain itself or may be a strain before the trait is changed by genetic variation due to natural or artificial factors. For example, the unmodified microorganism may refer to a strain in which the gluconate repressor variant described herein is not introduced or a strain before introduction thereof. The "unmodified microorganism" may be used interchangeably with "strain before being modified", "microorganism before being modified", "unvaried strain", "unmodified strain", "unvaried microorganism", or "reference microorganism".

The unmodified microorganism may be a microorganism comprising the amino acid sequence consisting of SEQ ID NO: 1 or the polynucleotide consisting of SEQ ID NO: 2.

The microorganism having the L-arginine-producing ability of the present disclosure may be a microorganism comprising any one or more of the variant of the present disclosure, the polynucleotide of the present disclosure, and the vector comprising the polynucleotide of the present disclosure; a microorganism modified to express the variant of the present disclosure or the polynucleotide of the present disclosure; a microorganism (e.g., recombinant strain) expressing the variant of the present disclosure or the polynucleotide of the present disclosure; or a microorganism (e.g., recombinant strain) having the activity of the variant of the present disclosure, but is not limited thereto.

For example, the microorganism of the present disclosure may be a cell or microorganism that is transformed with the polynucleotide of the present disclosure or the vector comprising the polynucleotide encoding the variant of the present disclosure to express the variant of the present disclosure. The microorganism of the present disclosure may comprise all microorganisms capable of producing L-arginine by comprising the variant of the present disclosure. For example, the strain of the present disclosure may be a recombinant strain with increased L-arginine-producing ability, in which the gluconate repressor variant is expressed by introducing the polynucleotide encoding the variant of the present disclosure into a natural wild-type microorganism or a microorganism with the L-arginine-producing ability. The recombinant strain with the increased L-arginine-producing ability may be a microorganism in which the L-arginine-producing ability is increased, as compared to a natural wild-type microorganism or a gluconate repressor-unmodified microorganism (e.g., a microorganism expressing the wild-type gluconate repressor or a microorganism not expressing the variant of the present disclosure), but is not limited thereto. For example, the strain with the increased L-arginine-producing ability of the present disclosure may be a microorganism in which the L-arginine-producing ability is increased, as compared to a microorganism comprising the polypeptide of SEQ ID NO: 1 or the polynucleotide encoding the same, but is not limited thereto.

The microorganism of the present disclosure may comprise all microorganisms capable of expressing the gluconate repressor variant of the present disclosure by various known methods, in addition to the introduction of the nucleic acid or vector.

For example, the microorganism with the increased L-arginine-producing ability may have an increased L-arginine-producing ability of about 1% or more, specifically, about 1% or more, about 2.5% or more, about 5% or more, about 6% or more, about 7% or more, about 8% or more, about 9% or more, about 10% or more, about 10.5% or more, about 11% or more, about 11.5%or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, or about 19% or more (the upper limit is not particularly limited, but may be, for example, about 200% or less, about 150% or less, about 100% or less, about 50% or less, about 45% or less, about 40% or less, about 35% or less, about 30% or less, or about 25% or less), as compared to that of the parent strain before modification or an unmodified microorganism having the endogenous activity of the gluconate repressor protein, but the increased amount is not limited thereto as long as the producing ability has an increased amount of a + value, as compared to that of the parent strain before modification or the unmodified microorganism. In another example, the recombinant strain with the increased production ability may have an increased L-arginine-producing ability of about 1.1 times or more, about 1.12 times or more, about 1.13 times or more, about 1.14 times or more, about 1.15 times or more, about 1.16 times or more, about 1.17 times or more, about 1.18 times or more, or about 1.19 times or more (the upper limit is not particularly limited, but may be, for example, about 10 times or less, about 5 times or less, about 3 times or less, about 2 times or less, about 1.5 times or less or about 1.3 times or less), as compared to that of the parent strain before modification or the unmodified microorganism, but is not limited thereto. The term "about" refers to a range which comprises all of ±0.5, ±0.4, ±0.3, ±0.2, ±0.1, etc. and comprises all of the values that are equivalent or similar to those following the values, but the range is not limited thereto.

The microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Specifically, the microorganism may be *Corynebacterium glutamicum,* but is not limited thereto.

Meanwhile, it has already been known that microorganisms of the genus *Corynebacterium* are able to produce L-arginine, but their productivity is significantly low, and the genes involved in the production mechanisms or the mechanisms have not been fully elucidated. Therefore, the microorganism of the genus *Corynebacterium* having the L-arginine-producing ability of the present disclosure may comprise any of a natural wild-type microorganism itself, a microorganism of the genus *Corynebacterium* having improved L-arginine-producing ability by strengthening or weakening the activity of genes related to the L-arginine production mechanism, or a microorganism of the genus *Corynebacterium* having improved L-arginine-producing ability by introducing or strengthening the activity of a foreign gene.

Still another aspect of the present disclosure provides a method of producing L-arginine, the method comprising the step of culturing the microorganism of the present disclosure in a medium.

Specifically, the method of producing L-arginine of the present disclosure may comprise the step of culturing, in a medium, the microorganism of the genus *Corynebacterium* comprising the variant of the present disclosure, the polynucleotide of the present disclosure, or the vector of the present disclosure, but is not limited thereto.

As used herein, the term "culturing" refers to growing the microorganism of the present disclosure in appropriately adjusted environment conditions. The culture procedure of the present disclosure may be performed according to appropriate media or culture conditions known in the art. Such culture procedure may be easily adjusted according to the selected strain by a person skilled in the art. Specifically, the culture may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the microorganism of the present disclosure, any medium that is used for usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

For example, the culture medium for the strain of the genus *Corynebacterium* may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, the pH of the culture may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-comprising gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to about 160 hours, but is not limited thereto.

The L-arginine produced by the culturing of the present disclosure may be released into the medium or may remain in cells.

The method of producing L-arginine of the present disclosure may further comprise the steps of preparing the microorganism of the present disclosure, preparing a medium for culturing the microorganism, or a combination thereof (regardless of the order, in any order), for example, before the culturing step.

The method of producing L-arginine of the present disclosure may further comprise the step of recovering the L-arginine from a medium resulting from the culturing (a medium in which culturing has been performed) or the microorganism of the present disclosure. The recovering step may be further comprised after the culturing step.

The recovering may be collecting L-arginine by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and L-arginine may be recovered from the medium or microorganism by using an appropriate method known in the art.

In addition, the method of producing L-arginine of the present disclosure may further comprise a purification step. The purification may be performed by using an appropriate method known in the art. In an exemplary embodiment, when the method of producing L-arginine of the present disclosure comprises both the recovering step and the purification step, the recovering step and the purification step may be performed continuously or discontinuously regardless of the order, or may be performed simultaneously or integrated into one step, but is not limited thereto.

Still another aspect of the present disclosure provides a composition for producing L-arginine, the composition comprising the microorganism comprising any one or more of the gluconate repressor variant of the present disclosure, the polynucleotide encoding the variant, and the vector comprising the polynucleotide; a culture of the microorganism; or a combination of two or more thereof.

The composition of the present disclosure may further comprise any appropriate excipient that is usually used in the composition for producing amino acids, and such excipients may comprise, for example, a preserving agent, a wetting agent, a dispersing agent, a suspending agent, a buffering agent, a stabilizing agent, an isotonic agent, etc., but are not limited thereto.

Still another aspect of the present disclosure provides use of the microorganism of the present disclosure in producing L-arginine.

Still another aspect of the present disclosure provides use of the gluconate repressor variant in producing L-arginine, the gluconate repressor variant having substitutions of one or more amino acids selected from the group consisting of the amino acid corresponding to position 36, the amino acid corresponding to position 59, the amino acid corresponding to position 60, the amino acid corresponding to position 63, the amino acid corresponding to position 79, and the amino acid corresponding to position 92 of the amino acid sequence of SEQ ID NO: 1 with different amino acids.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Construction of gluconate repressor (gntR1) mutation vector

To insert gntR1(136N), gntR1(R59A), gntR1(T60L), gntR1(R63A), gntR1(E70K), gntR1(R79A), and gntR1(A92L) mutations, each represented by SEQ ID NO: 23 to SEQ ID NO: 29, into a *Corynebacterium glutamicum* KCCM10741P strain, vectors comprising each target mutation were constructed. In detail, PCR was performed using genomic DNA of *Corynebacterium glutamicum* ATCC13869 as a template and primer pairs of Table 1 (SEQ ID NOS: 3 and 5, SEQ ID NOS: 4 and 6, SEQ ID NOS: 3 and 7, SEQ ID NOS: 4 and 8, SEQ ID NOS: 3 and 9, SEQ ID NOS: 4 and 10, SEQ ID NOS: 3 and 11, SEQ ID NOS: 4 and 12, SEQ ID NOS: 3 and 13, SEQ ID NOS: 4 and 14, SEQ ID NOS: 3 and 15, SEQ ID NOS: 4 and 16, SEQ ID NOS: 3 and 17, SEQ ID NOS: 4 and 18), and overlapping PCR was performed using primer pairs of SEQ ID NOS: 3 and 4 to obtain homologous recombination fragments (hereinbelow, "mutation-introduced fragment 1" to "mutation-introduced fragment 7"), each having gntR1-I36N, R59A, T60L, R63A, E70K, R79A, or A92L mutation sequence. At this time, the PCR reaction was performed by repeating 30 cycles of denaturation at 95°C for 30 seconds; annealing at 55°C for 30 seconds; and extension at 72°C for 1 minute.

Subsequently, the pDCM2 (Korean Patent Publication No. 10-2020-0136813) vector that is not replicable in *Corynebacterium glutamicum* and the above fragments amplified by PCR, were treated with BamHI and Xbal, which are restriction enzymes for chromosomal insertion, and then ligated using DNA ligase to prepare plasmids into which each mutation-introduced fragment was inserted, and each plasmid was transformed into *E.coli* DH5α, which was plated on LB solid medium comprising kanamycin (25 mg/l). Colonies transformed with the plasmid were selected from the LB solid medium, and then each DNA was obtained using a DNA-spin plasmid DNA purification kit (iNtRON), thereby preparing pDCM2-gntR1(136N), pDCM2-gntR1(R59A), pDCM2-gntR1(T60L), pDCM2-gntR1(R63A), pDCM2-gntR1(E70K), pDCM2-gntR1(R79A), and pDCM2-gntR1(A92L) vectors (recombinant plasmids), each comprising the mutation-introduced fragment 1 to mutation-introduced fragment 7.

**[Table 1]**

| No. | Name | DNA sequence (5'-3') |
|---|---|---|
| SEQ ID NO: 3 | gntR1-AF | tcggtacccggggatccAAGCAACCGTCGG CGAG |
| SEQ ID NO: 4 | gntR1-BR | tgcaggtcgactctagaTTTCGGGACGCAGT TTC |
| SEQ ID NO: 5 | gntR1(I36N)-AR | AGCAACCTTGCCACTGtTGATATCTTG ACCGAGC |
| SEQ ID NO: 6 | gntR1(I36N)-BF | aCAGTGGCAAGGTTGCTGTCGGAGAT ACCTTCAAG |
| SEQ ID NO: 7 | gntR1(R59A)-AR | TCGCGTGCCACTGTGgcGGAAATACC AAAACGC |
| SEQ ID NO: 8 | gntR1(R59A)-BF | gcCACAGTGGCACGCGAAGCAATGCG CGCTTTG |
| SEQ ID NO: 9 | gntR1(T60L)-AR | GCTTCGCGTGCCACTagGCGGGAAAT ACCAAAACG |
| SEQ ID NO: 10 | gntR1(T60L)-BF | ctAGTGGCACGCGAAGCAATGCGCGC TTTGGAGCA |
| SEQ ID NO: 11 | gntR1(R63A)-AR | GCGCGCATTGCTTCGgcTGCCACTGT GCGGG |
| SEQ ID NO: 12 | gntR1(R63A)-BF | gcCGAAGCAATGCGCGCTTTGGAGCA GCTCGGTC |
| SEQ ID NO: 13 | gntR1(E70K)-AR | CGAGCTGcttCAAAGCGCGCATTGCTT CGCGTGC |
| SEQ ID NO: 14 | gntR1(E70K)-BF | CGCTTTGaagCAGCTCGGTCTTGTCG CTTCTTCAC |
| SEQ ID NO: 15 | gntR1(R79A)-AR | GTAATGCCAATGCGAgcTGAAGAAGC GACAAGACC |
| SEQ ID NO: 16 | gntR1(R79A)-BF | gcTCGCATTGGCATTACTGTTTTGCCA CAGGAAG |
| SEQ ID NO: 17 | gntR1(A92L)-AR | GACTTATCAAAAACAagCCACTCTTCC TGTGGC |
| SEQ ID NO: 18 | gntR1(A92L)-BF | ctTGTTTTTGATAAGTCCATCATTCGCT GGCGTC |

### Example 2. Preparation of strain comprising gluconate repressor (gntR1) variant based on KCCM10741P strain and Evaluation of L-arginine-producing ability

### Example 2-1. Preparation of strain

To introduce mutations into the *gntR1* gene of *Corynebacterium glutamicum* KCCM10741P (US 8034602 B2), which is an L-arginine-producing strain, the recombinant plasmids (pDCM2-gntR1(I36N), pDCM2-gntR1(R59A), pDCM2-gntR1(T60L), pDCM2-gntR1(R63A), pDCM2-gntR1(E70K), pDCM2-gntR1(R79A), and pDCM2-gntR1(A92L)) prepared in Example 1 were respectively used to perform transformation (van der Rest et al., Appl Microbiol Biotechnol 52:541-545, 1999). Subsequently, secondary recombination was performed on a solid plate comprising 4% sucrose, and PCR was performed using a primer pair (SEQ ID NOS: 3 and 4) on the transformants, in which secondary recombination was completed, to confirm that each mutation was introduced into the gntR1 gene on the chromosome. At this time, the PCR reaction was performed by repeating 30 cycles of denaturation at 95°C for 30 seconds; annealing at 55°C for 30 seconds; and extension at 72°C for 1 minute.

The transformants were named KCCM10741P-gntR1(136N), KCCM10741P-gntR1(R59A), KCCM10741P-gntR1(T60L), KCCM10741P-gntR1(R63A), KCCM10741P-gntR1(E70K), KCCM10741P-gntR1(R79A), and KCCM10741P-gntR1(A92L), respectively.

### Example 2-2. Evaluation of L-arginine-producing ability

To compare L-arginine-producing ability between the L-arginine-producing strain *Corynebacterium glutamicum* KCCM10741P, and the transformants prepared in Example 2-1 (gntR1 variant-introduced strains, each KCCM10741P-gntR1(136N), KCCM10741P-gntR1(R59A), KCCM10741P-gntR1(T60L), KCCM10741P-gntR1(R63A), KCCM10741P-gntR1(E70K), KCCM10741P-gntR1(R79A), and KCCM10741P-gntR1(A92L)), the strains were cultured by the following method, and the concentration of L-arginine in the culture was analyzed.

The parent strain *Corynebacterium glutamicum* KCCM10741P and the transformed strains (strains into which each gntR1 variant was introduced) prepared in Example 2-1 were respectively inoculated into a 250-mL corner-baffled flask comprising 25 mL of the following seed medium, followed by culture with shaking at 30°C for 20 hours at 200 rpm. Then, 1 mL of each seed culture was inoculated into a 250-mL corner-baffled flask comprising 24 mL of a production medium, followed by culture with shaking at 30°C for 72 hours at 200 rpm. The compositions of the seed medium and the production medium are as follows, respectively.

### <Seed medium (pH 7.2)>

20 g of glucose, 45 g of ammonium sulfate, 2 g of magnesium sulfate heptahydrate, 2 g of potassium phosphate monobasic, 10 g of ammonium chloride, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate (based on 1 liter of distilled water)

### <Production medium (pH 7.2)>

60 g of glucose, 45 g of ammonium sulfate, 2 g of magnesium sulfate heptahydrate, 2 g of potassium phosphate monobasic, 10 g of ammonium chloride, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate, 30 g of calcium carbonate (based on 1 liter of distilled water)

After completing the culture, the L-arginine-producing ability (concentration) was analyzed using HPLC (Waters 2478), and the analyzed L-arginine concentrations are shown in Table 2 below.

**[Table 2]**

| | Name of strain | L-arginine (g/L) | | | |
|---|---|---|---|---|---|
| | | Batch 1 | Batch 2 | Batch 3 | Mean |
| Control group | KCCM10741P | 2.9 | 3.0 | 3.1 | 3.0 |
| Experimental group | KCCM10741P-gntR1(I36N) | 3.4 | 3.6 | 3.5 | 3.5 |
| | KCCM10741P-gntR1(R59A) | 3.6 | 3.3 | 3.5 | 3.5 |
| | KCCM10741P-gntR1(T60L) | 3.4 | 3.5 | 3.5 | 3.5 |
| | KCCM10741P-gntR1(R63A) | 3.6 | 3.5 | 3.0 | 3.4 |
| | KCCM10741P-gntR1(E70K) | 3.6 | 3.8 | 3.2 | 3.5 |
| | KCCM10741P-gntR1(R79A) | 3.4 | 3.7 | 3.5 | 3.5 |
| | KCCM10741P-gntR1(A92L) | 3.6 | 3.5 | 3.3 | 3.5 |

As a result, it was confirmed that L-arginine producing ability increased by about 17% on average in all of the transformants (gntR1 variant-introduced strains, each KCCM10741P-gntR1(I36N), KCCM10741P-gntR1(R59A), KCCM10741P-gntR1(T60L), KCCM10741P-gntR1(R63A), KCCM10741P-gntR1(E70K), KCCM10741P-gntR1(R79A), and KCCM10741P-gntR1(A92L)), as compared to the parent strain.

### Example 3. Preparation of strain comprising gluconate repressor (gntR1) variant based on CJ1R strain and Evaluation of L-arginine-producing ability

### Example 3-1. Preparation of CJ1R strain

To examine whether the effect of increasing L-arginine-producing ability is also exhibited in other *Corynebacterium glutamicum* strains having the L-arginine-producing ability, one type of mutation (△argR) was introduced into the wild-type (ATCC13869) to further prepare *Corynebacterium glutamicum* strain CJ1R having L-arginine-producing ability. In detail, PCR was performed using genomic DNA of *Corynebacterium glutamicum* ATCC13869 as a template and primer pairs of Table 3 (SEQ ID NOS: 19 and 20, SEQ ID NOS: 21 and 22), and overlapping PCR was performed using primer pairs of SEQ ID NOS: 19 and 22 to obtain a homologous recombination fragment having argR deletion mutation sequence. At this time, the PCR reaction was performed by repeating 30 cycles of denaturation at 95°C for 30 seconds; annealing at 55°C for 30 seconds; and extension at 72°C for 1 minute. The prepared vector (recombinant plasmid) was named pDCM2-△argR.

**[Table 3]**

| No. | Name | DNA sequence (5'-3') |
|---|---|---|
| SEQ ID NO: 19 | argR-AF | tcggtacccggggatccAGCAGGCCTTAAGGGTA AG |
| SEQ ID NO: 20 | argR-AR | AGGGGCGCTGTCTTACCTCGGCTGGTGG GCCAGC |
| SEQ ID NO: 21 | argR-BF | GGTAAGACAGCGCCCCTAGTTCAAGGCTT GTTAATC |
| SEQ ID NO: 22 | argR-BR | tgcaggtcgactctagaACCGTTGAACTGCTTGC CAG |

Subsequently, pDCM2-△argR prepared above was transformed into the wild-type *Corynebacterium glutamicum* (ATCC13869) (van der Rest et al., Appl Microbiol Biotechnol 52:541-545, 1999), and then secondary recombination was performed on a solid plate comprising 4% sucrose, and PCR was performed using a primer pair (SEQ ID NOS: 19 and 22) on the transformant, in which secondary recombination was completed, to identify the strain in which argR on the chromosome was deleted. At this time, the PCR reaction was performed by repeating 30 cycles of denaturation at 95°C for 30 seconds; annealing at 55°C for 30 seconds; and extension at 72°C for 1 minute. The transformed strain was named CJ1R.

### Example 3-2. Preparation of strain comprising gluconate repressor (gntR1) variant based on CJ1R strain

To introduce gntR1 mutation into the CJ1R strain, mutant strains, into which each of the vectors prepared in Example 1 was introduced, were prepared in the same manner as in Example 2-1, and the mutant strains were named CJ1R-gntR1(136N), CJ1R-gntR1(R59A), CJ1R-gntR1(T60L), CJ1R-gntR1(R63A), CJ1R-gntR1(E70K), CJ1R-gntR1(R79A), and CJ1R-gntR1(A92L), respectively.

### Example 3-3. Evaluation of L-arginine-producing ability

To compare the L-arginine-producing ability between the L-arginine-producing strain *Corynebacterium glutamicum* CJ1R, and the mutant strains prepared in Example 3-2 (gntR1 variant-introduced strains, each CJ1R-gntR1(136N), CJ1R-gntR1(R59A), CJ1R-gntR1(T60L), CJ1R-gntR1(R63A), CJ1R-gntR1(E70K), CJ1R-gntR1(R79A), and CJ1R-gntR1(A92L)), the strains were cultured in the same manner as in Example 2-2, and the L-arginine concentrations in the cultures were analyzed.

After completing the culture, the L-arginine-producing ability (concentration) was analyzed using HPLC (Waters 2478), and the analyzed L-arginine concentrations are shown in Table 4 below.

**[Table 4]**

| | Name of strain | L-arginine (g/L) | | | |
|---|---|---|---|---|---|
| | | Batch 1 | Batch 2 | Batch 3 | Mean |
| Control group | CJ1R | 2.0 | 2.2 | 2.1 | 2.1 |
| Experimental group | CJ1R-gntR1(I36N) | 2.5 | 2.7 | 2.5 | 2.6 |
| | CJ1R-gntR1(R59A) | 2.3 | 2.6 | 2.8 | 2.6 |
| | CJ1R-gntR1(T60L) | 2.6 | 2.5 | 2.4 | 2.5 |
| | CJ1R-gntR1(R63A) | 2.4 | 2.4 | 2.6 | 2.5 |
| | CJ1R-gntR1(E70K) | 2.7 | 2.4 | 2.5 | 2.5 |
| | CJ1R-gntR1(R79A) | 2.5 | 2.6 | 2.7 | 2.6 |
| | CJ1R-gntR1(A92L) | 2.5 | 2.5 | 2.3 | 2.4 |

As a result, it was confirmed that L-arginine producing ability increased by about 19% on average in all of the mutant strains (gntR1 variant-introduced strains, each CJ1R-gntR1(I36N), CJ1R-gntR1(R59A), CJ1R-gntR1(T60L), CJ1R-gntR1(R63A), CJ1R-gntR1(E70K), CJ1R-gntR1(R79A), and CJ1R-gntR1(A92L)), as compared to the parent strain.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A gluconate repressor variant, wherein one or more amino acids selected from the group consisting of an amino acid corresponding to position 36, an amino acid corresponding to position 59, an amino acid corresponding to position 60, an amino acid corresponding to position 63, an amino acid corresponding to position 79, and an amino acid corresponding to position 92 of an amino acid sequence of SEQ ID NO: 1 are substituted with different amino acids.

2. The gluconate repressor variant of claim 1, wherein an amino acid corresponding to position 70 of the amino acid sequence of SEQ ID NO: 1 is further substituted with a different amino acid.

3. The gluconate repressor variant of claim 1, comprising one or more substitutions selected from the group consisting of a substitution of the amino acid corresponding to position 36 with asparagine, a substitution of the amino acid corresponding to position 59 with alanine, a substitution of the amino acid corresponding to position 60 with leucine, a substitution of the amino acid corresponding to position 63 with alanine, a substitution of the amino acid corresponding to position 79 with alanine, and a substitution of the amino acid corresponding to position 92 with leucine in the amino acid sequence of SEQ ID NO: 1.

4. The gluconate repressor variant of claim 2, wherein the different amino acid is lysine.

5. The gluconate repressor variant of claim 1, wherein the gluconate repressor variant has 80% or more sequence identity to SEQ ID NO: 1.

6. A polynucleotide encoding the gluconate repressor variant of any one of claims 1 to 5.

7. A microorganism of the genus *Corynebacterium,* comprising the gluconate repressor variant of any one of claims 1 to 5 or a polynucleotide encoding the variant.

8. The microorganism of the genus *Corynebacterium* of claim 7, wherein the microorganism has L-arginine-producing ability.

9. The microorganism of the genus *Corynebacterium* of claim 7, wherein the microorganism of the genus *Corynebacterium* is *Corynebacterium glutamicum.*

10. A microorganism of the genus *Corynebacterium* comprising a gluconate repressor variant having a substitution of an amino acid corresponding to position 70 of an amino acid sequence of SEQ ID NO: 1 with a different amino acid, or a polynucleotide encoding the variant, wherein the microorganism of the genus *Corynebacterium* has L-arginine-producing ability.

11. The microorganism of the genus *Corynebacterium* of claim 10, wherein the microorganism of the genus *Corynebacterium* is *Corynebacterium glutamicum.*

12. A method of producing L-arginine, the method comprising the step of culturing the microorganism of the genus *Corynebacterium* of claim 7 in a medium.

13. A method of producing L-arginine, the method comprising the step of culturing the microorganism of the genus *Corynebacterium* of claim 10 in a medium.
